(19) Europäisches Patentamt
European Patent Office
Office européen des brevets

(11) **EP 3 786 175 A1**

(12) **EUROPEAN PATENT APPLICATION**
published in accordance with Art. 153(4) EPC

(43) Date of publication:
**03.03.2021 Bulletin 2021/09**

(21) Application number: **19793469.8**

(22) Date of filing: **07.05.2019**

(51) Int Cl.:
*C07K 14/47* (2006.01)   *C12N 5/071* (2010.01)

(86) International application number:
**PCT/JP2019/018271**

(87) International publication number:
**WO 2019/208831 (31.10.2019 Gazette 2019/44)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **27.04.2018 JP 2018087321**

(71) Applicants:
• **Toppan Printing Co., Ltd.**
**Tokyo 110-0016 (JP)**

• **Osaka University**
**Suita-shi, Osaka 565-0871 (JP)**

(72) Inventors:
• **KITANO, Shiro**
**Tokyo 110-0016 (JP)**
• **IRIE, Shinji**
**Tokyo 110-0016 (JP)**
• **MATSUSAKI, Michiya**
**Suita-shi, Osaka 565-0871 (JP)**

(74) Representative: **TBK**
**Bavariaring 4-6**
**80336 München (DE)**

(54) **EXTRACELLULAR-MATRIX-CONTAINING COMPOSITION, TEMPORARY SCAFFOLD FOR THREE-DIMENSIONAL TISSUE FORMATION, THREE-DIMENSIONAL TISSUE FORMATION AGENT, AND METHOD FOR RECOVERING CELLS FROM THREE-DIMENSIONAL TISSUE**

(57)    The present invention relates to an extracellular-matrix-containing composition comprising: a fragmented extracellular matrix component; and an aqueous medium.

## Description

## Technical Field

The present invention relates to an extracellular-matrix-containing composition, a temporary scaffold for three-dimensional tissue construct formation, and a three-dimensional tissue construct formation agent. The present invention also relates to a method for recovering a cell from a three-dimensional tissue construct.

## Background Art

Techniques to construct a three-dimensional tissue construct of cells ex vivo have been developed in recent years. Proposed are, for example, a method of producing a three-dimensional tissue construct by culturing coated cells, which are cultured cells whose whole surfaces are each covered with an adhesion film (Patent Literature 1), and a method of producing a three-dimensional tissue construct by seeding cells on a scaffold made of polylactic acid or the like (Non Patent Literature 1). The present inventors have previously proposed, for example, a method of producing a three-dimensional tissue construct, comprising: forming a three-dimensional tissue construct by three-dimensionally disposing cells each coated with a coating containing an extracellular matrix component such as a collagen component and a fibronectin component (Patent Literature 2), and a method of producing a three-dimensional tissue construct, comprising: forming coated cells with a coating formed on the surface of each cell; and three-dimensionally disposing the coated cells, wherein forming coated cells comprises: soaking cells in a solution containing a coating component; and separating the soaked cells and the solution containing the coating component by using a liquid-permeable membrane (Patent Literature 3). Such three-dimensional tissue constructs are expected to be applicable to alternatives for experimental animals, materials for transplantation, and so forth.

Various techniques have been examined, such as techniques using a scaffold material that allows cells to adhere thereto as described above, and techniques of stacking cells without use of a scaffold material, and culture of cells under coexistence with an extracellular matrix component such as a collagen component is commonly performed in any of the cell culture techniques. This is because such an extracellular matrix component functions as a material to physically support the tissue structure in the intercellular matrix, and is in addition inferred to play a biologically important role in cell development, differentiation, morphogenesis, and so forth.

## Citation List

## Patent Literature

Patent Literature 1: Japanese Unexamined Patent Publication No. 2012-115254
Patent Literature 2: International Publication No. WO 2015/072164
Patent Literature 3: International Publication No. WO 2016/027853
Patent Literature 4: Japanese Patent No. 6029102
Patent Literature 5: Japanese Patent No. 5870408

## Non Patent Literature

Non Patent Literature 1: Nature Biotechnology, 2005, Vol. 23, NO. 7, p. 879-884

## Summary of Invention

## Problems to be Solved by the Invention

In culturing cells with feeding an extracellular matrix component from the outside, it follows that a three-dimensional tissue construct constructed inevitably contains the extracellular matrix component fed from the outside (exogenous extracellular matrix component). Coexistence of an exogenous extracellular matrix component and cells is useful in a certain aspect in the stage of three-dimensionally culturing cells in vitro, whereas the exogenous extracellular matrix component, which is originally absent in a natural state, may affect the original functions of a constructed three-dimensional tissue construct in the stage of performing various analyses, examinations, evaluations, and so forth, for the three-dimensional tissue construct.

For example, a method of removing only exogenous components after formation of a three-dimensional tissue construct is contemplated as a method for avoiding such influence. In Patent Literature 4, for example, a carrier material is used that exhibits thermally reversible sol-gel transition such that the carrier material is converted into a gel state at a temperature of 25°C or more and into a sol state at a temperature of 0°C or more and 15°C or less. In Patent Literature 4, a technique is performed in which a three-dimensional tissue construct is formed on a carrier material after undergoing gelation and thereafter the carrier material is cooled to undergo solation, and the carrier material after undergoing solation is removed to recover the three-dimensional tissue construct.

However, a matter of concern for the technique according to Patent Literature 4 is influence on a three-dimensional tissue construct in the culturing process because the carrier material that exhibits thermally reversible sol-gel transition is an artificially produced material. In addition, a collagen component is separately loaded as an exogenous extracellular matrix component in Patent Literature 4, and hence it follows that the exogenous extracellular matrix component is still contained in a three-dimensional tissue construct recovered.

If a collagen component is loaded as an exogenous extracellular matrix component, for example, the exogenous collagen component can be dissolved by treating with collagenase to decompose collagen; however, such treatment may unexpectedly damage the tissue structure based on cells themselves and an endogenous collagen component secreted from the cells.

The present invention was made in view of the above circumstances, and an object of the present invention is to provide an extracellular-matrix-containing composition that exhibits thermally reversible sol-gel transition.

**Means for Solving the Problems**

The present inventors diligently studied to find that the problems are successfully solved through the invention shown below.

Specifically, the present invention provides, for example, (1) to (11) in the following.

(1) An extracellular-matrix-containing composition comprising:

a fragmented extracellular matrix component; and
an aqueous medium.

(2) The extracellular-matrix-containing composition according to (1), wherein the fragmented extracellular matrix component comprises a fragmented collagen component.
(3) The extracellular-matrix-containing composition according to (1) or (2), wherein at least a part of the fragmented extracellular matrix component is fibrillar.
(4) The extracellular-matrix-containing composition according to any one of (1) to (3), wherein an average length of the fragmented extracellular matrix component is 100 nm to 400 $\mu$m.
(5) The extracellular-matrix-containing composition according to any one of (1) to (4), wherein a content of the extracellular matrix component is 1 mg/mL or more and 100 mg/mL or less based on a total amount of the extracellular-matrix-containing composition.
(6) The extracellular-matrix-containing composition according to any one of (1) to (5), wherein the fragmented extracellular matrix component is naturally-occurring.
(7) The extracellular-matrix-containing composition according to any one of (1) to (6), wherein the extracellular-matrix-containing composition undergoes gelation from a sol state at 35.5°C $\pm$ 2°C, and undergoes solation from a gel state at 4.5°C $\pm$ 2°C.
(8) A temporary scaffold for three-dimensional tissue construct formation, the temporary scaffold comprising: the extracellular-matrix-containing composition according to any one of (1) to (7).
(9) A three-dimensional tissue construct formation agent with a high content ratio of an extracellular matrix component, the three-dimensional tissue construct formation agent comprising: the extracellular-matrix-containing composition according to any one of (1) to (7).
(10) A three-dimensional tissue construct comprising: the extracellular-matrix-containing composition according to any one of (1) to (7).
(11) A method for recovering a cell from a three-dimensional tissue construct comprising a fragmented extracellular matrix component and a cell, comprising:

a step of cooling the three-dimensional tissue construct to allow the fragmented extracellular matrix component to undergo solation; and
a step of removing the fragmented extracellular matrix component after undergoing solation.

**Effects of the Invention**

According to the present invention, an extracellular-matrix-containing composition that exhibits thermally reversible sol-gel transition can be provided. A three-dimensional tissue construct formed by using the extracellular-matrix-containing composition of the present invention allows exogenous extracellular matrix components to undergo solation on being cooled after the lapse of a certain period from initiation of culture, and hence exogenous extracellular matrix components can be removed from the three-dimensional tissue construct without damaging the three-dimensional tissue construct itself.

**Brief Description of Drawings**

[Figure 1] Figure 1 shows a microphotograph of a fragmented collagen component.
[Figure 2] Figure 2 shows a graph representing measurement results of transmittance at 500 nm for compositions containing a fragmented collagen component and phosphate-buffered saline at 4°C.
[Figure 3] Figure 3 (A) shows a graph representing measurement results of transmittance at 500 nm for fragmented-collagen-containing solutions and a commercially available collagen-containing solution at 37°C, and Figure 3 (B) shows a graph representing measurement results of transmittance at 500 nm for fragmented-collagen-containing solutions and a commercially available collagen-containing solution at 4°C.
[Figure 4] Figure 4 shows photographs of a fragmented-collagen-containing solution at 4°C or 37°C.
[Figure 5] Figure 5 (A) shows photographs of a commercially available collagen-containing solution at 37°C and 4°C, and Figure 5 (B) shows photographs of a fragmented-collagen-containing solution at 37°C and 4°C.
[Figure 6] Figures 6 (A) to (C) show microphotographs of three-dimensional tissue constructs including a fragmented collagen component and cells.
[Figure 7] Figures 7 (A) and (B) show microphotographs demonstrating results of cell recovery.
[Figure 8] Figure 8 shows a graph representing sol-gel transition of solutions each containing 2% by mass, 3% by mass, or 5% by mass of a fragmented collagen component.
[Figure 9] Figures 9 (A) to (C) show photographs respectively representing sol-gel transition of pig-derived, bovine-derived, and human-derived fragmented-collagen-containing solutions.
[Figure 10] Figure 10 shows a graph representing results of CD spectrum measurement for a fragmented collagen component.
[Figure 11] Figure 11 shows photographs demonstrating results of analysis with SDS-PAGE for a fragmented collagen component.

**Embodiments for Carrying Out the Invention**

Hereinafter, modes for implementing the present invention will be described in detail. However, the present invention is not limited to the following embodiments.

<Extracellular-Matrix-Containing Composition>

The extracellular-matrix-containing composition according to the present embodiment comprises: a fragmented extracellular matrix component; and an aqueous medium. The extracellular-matrix-containing composition according to the present embodiment exhibits thermally reversible sol-gel transition.
In forming a three-dimensional tissue construct by using a collagen component as an exogenous extracellular matrix component (exogenous collagen component), for example, the gelation of the exogenous collagen component proceeds when culture is performed under physiological conditions. The exogenous collagen component that has gelled functions as a support to which cells adhere in the initial stage of three-dimensional culture, and simultaneously has biological influence on cells. It is known from experimental facts that such gelation of exogenous collagen components under physiological conditions is generally thermally irreversible (e.g., Patent Literature 5). For this reason, it is normally not easy to remove only an exogenous collagen component after culture for a certain period from the occurrence of gelation. By contrast, the extracellular-matrix-containing composition according to the present embodiment undergoes solation through cooling even after gelation has once occurred, and hence exogenous extracellular matrix components can be removed after formation of a three-dimensional tissue construct. Thereby, a three-dimensional tissue construct from which exogenous extracellular matrix components have been partially or totally removed can be recovered.
The extracellular matrix component, formed of multiple extracellular matrix molecules, is an assembly of extracellular matrix molecules. Extracellular matrix molecules refer to substances present out of cells in living organisms.

Any substance as an extracellular matrix molecule may be used unless an adverse effect is caused on the growth of cells and formation of a cell assembly. Examples of extracellular matrix molecules include, but are not limited to, collagen, laminin, fibronectin, vitronectin, elastin, tenascin, entactin, fibrillin, and proteoglycan. One of these extracellular matrix components may be used singly, and any combination of them may be used. Modified products and variants of the above-mentioned extracellular matrix molecules are acceptable unless an adverse effect is caused on the growth of cells and formation of a cell assembly.

Examples of collagen include fibrillar collagen and non-fibrillar collagen. Fibrillar collagen refers to collagen that serves as a main component of collagen fibers, and specific examples thereof include type I collagen, type II collagen, and type III collagen. Examples of non-fibrillar collagen include type IV collagen.

Examples of proteoglycan include, but are not limited to, chondroitin sulfate proteoglycan, heparan sulfate proteoglycan, keratan sulfate proteoglycan, and dermatan sulfate proteoglycan.

The extracellular matrix component may contain at least one selected from the group consisting of collagen, laminin, and fibronectin, and it is preferable that the extracellular matrix component contain collagen. The collagen is preferably fibrillar collagen, and more preferably type I collagen. Commercially available collagen components may be used for the fibrillar collagen, and specific examples thereof include a freeze-dried product of porcine skin collagen type I produced by NH Foods Ltd. It is preferable that the collagen be atelocollagen, a collagen removed of telopeptide. Atelocollagen can be obtained, for example, through pepsin treatment of tropocollagen. It is preferable that the collagen is atelocollagen because the extracellular-matrix-containing composition exhibits more superior thermoresponsivity.

The extracellular matrix component may be an animal-derived extracellular matrix component. The animal species from which the extracellular matrix component is derived may be, for example, a mammalian species, an avian species, a reptilian species, or a fish species, and it is preferable that the animal species from which the extracellular matrix component is derived be a mammalian species. Examples of the animal species from which the extracellular matrix component is derived include, but are not limited to, humans, pigs, and bovines. The animal species from which the extracellular matrix component is derived may be a mammalian species, and it is preferable that the animal species from which the extracellular matrix component is derived be a pig, because particularly excellent thermoresponsivity is provided. For the extracellular matrix component, a component derived from one animal may be used, and components derived from a plurality of animals may be used in combination. The animal species from which the extracellular matrix component is derived may be the same as or different from the origin of cells for formation of a three-dimensional tissue.

Fragmented extracellular matrix is a component finely fragmented by applying physical force to the above-described extracellular matrix component. It is preferable that the fragmented extracellular matrix component be a fibrillated extracellular matrix component obtained by fibrillating the extracellular matrix component without cleaving bonds of extracellular matrix molecules. If the fragmented extracellular matrix component is a fibrillated extracellular matrix component, the sol-gel transition ability is even more superior, and the fragmented extracellular matrix component can be more effectively used as a scaffold material.

The manner of fragmenting the extracellular matrix component is not limited to a particular method. For example, the extracellular matrix component may be fragmented (or fibrillated) by applying physical force with an ultrasonic homogenizer, a stirrer-type homogenizer, a high-pressure homogenizer, or the like. In using a stirrer-type homogenizer, the extracellular matrix component may be directly homogenized, or homogenized in an aqueous medium such as saline. The fragmented extracellular matrix component can be obtained in millimeter-size or nanometer-size by adjusting the duration of homogenization, the number of homogenizing operations, and so forth. Alternatively, the fragmented extracellular matrix component can be obtained by fragmenting through repetitive freezing and thawing.

One extracellular matrix component or a combination of a plurality of extracellular matrix components may be used for the extracellular matrix component from which the fragmented extracellular matrix component is derived.

It is preferable that the fragmented extracellular matrix component contain a fragmented collagen component.

The fragmented extracellular matrix component may be naturally-occurring. The fragmented extracellular matrix component that is naturally-occurring is a fragmented product of a natural extracellular matrix component, and components obtained by modifying the structure of a natural extracellular matrix molecule with chemical treatment are not included in the category of the fragmented extracellular matrix component that is naturally-occurring. Examples of the chemical treatment include hydrolysis with alkali treatment.

Examples of the shape of the fragmented extracellular matrix component include fibrillar shapes. A fibrillar shape refers to a shape composed of a filamentous extracellular matrix component or a shape composed of an assembly of a plurality of filamentous extracellular matrix components. For example, it is preferable that the fragmented collagen component retain the triple helix structure (fibrillar shape) derived from collagen. It is preferable that at least a part of the fragmented extracellular matrix component be fibrillar. It is preferable that the fragmented extracellular matrix component be a fragmented collagen component at least a part of which is fibrillar.

In one embodiment, it is preferable that the average length of the fragmented extracellular matrix component be 100 nm to 400 $\mu$m, and the average length of the fragmented extracellular matrix component is more preferably 5 $\mu$m to 400 $\mu$m, 10 $\mu$m to 400 $\mu$m, 22 $\mu$m to 400 $\mu$m, or 100 $\mu$m to 400 $\mu$m, because a thick tissue tends to form. In another embodiment, the average length of the fragmented extracellular matrix component may be 100 nm to 100 μm, and is preferably 100 nm to 50 μm, 100 nm to 30 μm, 100 nm to 25 μm, 100 nm to 20 μm, 100 nm to 15 μm, 100 nm to 10 μm, or 100 nm to 1 μm. This case is preferred because tissue formation tends to be stable. It is preferable that the average length of most of all the fragmented extracellular matrix component be within the above numerical range. Specifically, it is preferable that the average length of 95% of all the fragmented extracellular matrix component be within the above numerical range. It is preferable that the fragmented extracellular matrix component be a fragmented collagen component whose average length is within the above range.

It is preferable that the average diameter of the extracellular matrix component be 50 nm to 30 μm, it is more preferable that the average diameter of the extracellular matrix component be 4 μm to 30 μm, and it is even more preferable that the average diameter of the extracellular matrix component be 20 μm to 30 μm. It is preferable that the fragmented extracellular matrix component be a fragmented collagen component whose average diameter is within the above range.

The average diameter and average length of the fragmented extracellular matrix component can be determined through measurement of individual parts of the fragmented extracellular matrix component with an optical microscope and subsequent image analysis. Herein, "average length" refers to an average value of lengths in the longitudinal direction of a sample under measurement, and "average diameter" refers to an average value of lengths in the direction perpendicular to the longitudinal direction of a sample under measurement.

The content of the fragmented extracellular matrix component is not limited to particular values, unless an adverse effect is caused on the growth of cells and formation of a cell assembly. To further enhance the effect to be expected as a scaffold for three-dimensional tissue construct formation and impart a larger thickness to a three-dimensional tissue construct to be formed, the content of the fragmented extracellular matrix component may be 0.01 mg/mL or more, 0.1 mg/mL or more, 1 mg/mL or more, 5 mg/mL or more, 10 mg/mL or more, 15 mg/mL or more, or 20 mg/mL or more, and 200 mg/mL or less, 100 mg/mL or less, 90 mg/mL or less, or 80 mg/mL or less based on the total amount of the extracellular-matrix-containing composition. It is preferable that the content of the fragmented extracellular matrix component be 1 mg/mL or more and 100 mg/mL or less based on the total amount of the extracellular-matrix-containing composition.

The "aqueous medium" refers to a liquid whose essential constituent component is water. The aqueous medium is not limited to a particular aqueous medium, as long as the aqueous medium allows the extracellular matrix component to stably exist therein. Examples of the aqueous medium include, but are not limited to, saline such as phosphate-buffered saline (PBS), and liquid culture media such as a Dulbecco's Modified Eagle's Medium (DMEM) and a liquid culture medium specialized for vascular endothelial cells (Endothelial Cell Growth Medium 2 (EGM2)). The aqueous medium may be an aqueous solution containing ethanol.

It is preferable that the pH of the aqueous medium be in such a range that an adverse effect is not caused on the growth of cells and formation of a cell assembly. To mitigate burdens on cells when the aqueous solution is loaded into the cells, the pH of the aqueous medium may be 7.0 or more, and may be 8.0 or less. The pH of the aqueous medium is, for example, 7.0, 7.1, 7.2, 7.3, 7.4, 7.5, 7.6, 7.7, 7.8, 7.9, or 8.0. It is preferable that the aqueous medium has buffer capacity in the above pH range, and the aqueous medium is more preferably a liquid culture medium. The liquid culture medium is not limited to a particular liquid culture medium, and a preferred culture medium may be selected according to the type of cells to be cultured. Examples of such culture media include an Eagle's MEM, a DMEM, a Modified Eagle's Medium (MEM), Minimum Essential Medium, an RPMI, and a GlutaMax Medium. The culture medium may be a medium with serum, or a serum-free medium. Further, the liquid culture medium may be a mixed culture medium obtained by mixing two or more culture media. The pH of the extracellular-matrix-containing composition may be the same as the pH of the above aqueous medium.

In using the extracellular-matrix-containing composition for three-dimensional tissue construct formation, it is preferable that the extracellular-matrix-containing composition be subjected to filtration sterilization in advance, and then stored or used for tissue formation. Extracellular matrix components (non-fragmented extracellular matrix components) such as collagen do not undergo sol-gel transition, and hence filtration sterilization through a sterilization filter is typically difficult when the concentration of such an extracellular matrix component is high. By contrast, the extracellular-matrix-containing composition according to the present embodiment is capable of being converted into a sol state through cooling, and hence allows filtration sterilization through a sterilization filter with ease.

The extracellular-matrix-containing composition according to the present embodiment is in a sol state at low temperature, and in a gel state at high temperature. For example, the extracellular-matrix-containing composition may undergo the progression of solation in a temperature range of at least 0°C or more and 15°C or less, and undergo the progression of gelation in a temperature range of at least 25°C or more. In the case that the aqueous medium is phosphate-buffered saline, the extracellular-matrix-containing composition may be in a gel state at 37°C and in a sol state at 4°C. Being in a sol state and being in a gel state can be confirmed by visual observation.

The temperature at which the extracellular-matrix-containing composition undergoes gelation from a sol state (gel transition temperature) may be, for example, 25 to 40°C, and is preferably 30 to 38°C and more preferably 33.5 to 37.5°C. The temperature at which the extracellular-matrix-containing composition undergoes solation from a gel state (sol transition temperature) may be, for example, 2 to 20°C, and is preferably 2 to 18°C and more preferably 2.5 to 6.5°C. The gel transition temperature is a temperature at which as the temperature of the extracellular-matrix-containing composition is gradually increased from 4°C to 40°C, the transmittance at 500 nm reaches an intermediate value of the maximum and minimum values. The rate of gradual temperature increase may be 0.5°C/min or 1°C/min. The sol transition temperature is a temperature at which as the temperature of the extracellular-matrix-containing composition is gradually decreased from 40°C to 4°C, the transmittance at 500 nm reaches an intermediate value of the maximum and minimum values. The rate of gradual temperature decrease may be 0.5°C/min or 1°C/min. The content of the fragmented extracellular matrix component in the extracellular-matrix-containing composition when the gel transition temperature and sol transition temperature are measured may be, for example, 2 to 5% by mass, 2% by mass, 3% by mass, or 5% by mass based on the total mass of the extracellular-matrix-containing composition.

The extracellular-matrix-containing composition may be one that undergoes gelation from a sol state at 35.5°C ± 2°C and undergoes solation from a gel state at 4.5°C ± 2°C.

Further, being in a sol state or in a gel state can be determined by measuring the transmittance at 500 nm. For example, determination can be made as being in a sol state if the transmittance at 500 nm is 40% or more, and as being in a gel state if the transmittance at 500 nm is less than 40%. The transmittance at 500 nm can be measured by using a method described later in Examples. The wavelength to measure the transmittance is not limited to 500 nm, and other wavelengths may be used. For example, the wavelength may be 550 nm, 600 nm, 650 nm, 700 nm, or 750 nm. The viscosity of a gel state is higher than that of a sol state.

<Uses of Extracellular-Matrix-Containing Composition>

The extracellular-matrix-containing composition according to the present embodiment can be preferably used as a temporary scaffold for forming three-dimensional tissue constructs (temporary scaffold for three-dimensional tissue construct formation). Accordingly, provided as one embodiment of the present invention is a temporary scaffold for three-dimensional tissue construct formation comprising the above-described extracellular-matrix-containing composition. The temporary scaffold refers to a scaffold removable after formation of a three-dimensional tissue construct. Here, "removable after formation of a three-dimensional tissue construct" means that at least a part of the fragmented extracellular matrix component in the extracellular-matrix-containing composition is removable.

The extracellular-matrix-containing composition according to the present embodiment can contain a high concentration of the fragmented extracellular matrix component (preferably, a fragmented collagen component). Specifically, the content ratio of the fragmented extracellular matrix component in the extracellular-matrix-containing composition can be 2% by mass or more. Hence, the extracellular-matrix-containing composition can be preferably used for production of a three-dimensional tissue construct with a high content ratio of an extracellular matrix component. That is, the extracellular-matrix-containing composition according to the present embodiment can be preferably used as a three-dimensional tissue construct formation agent with a high content ratio of an extracellular matrix component. Accordingly provided as one embodiment of the present invention is a three-dimensional tissue construct formation agent with a high content ratio of an extracellular matrix component comprising the above-described extracellular-matrix-containing composition.

The three-dimensional tissue construct with a high content ratio of an extracellular matrix component refers to a three-dimensional tissue construct in which the content ratio of an extracellular matrix component is 10% by mass or more based on the three-dimensional tissue construct. The content ratio of an extracellular matrix component in the three-dimensional tissue construct may be 30% by mass or more, 40% by mass or more, or 50% by mass or more based on the three-dimensional tissue construct. The content ratio of an extracellular matrix component is a content ratio of an extracellular matrix component including the fragmented extracellular matrix component. The content ratio of an extracellular matrix component can be calculated from the volume of the three-dimensional tissue construct and the mass of the decellularized three-dimensional tissue construct.

<Three-dimensional tissue construct>

The three-dimensional tissue construct according to one embodiment comprises the above-described extracellular-matrix-containing composition. The three-dimensional tissue construct further comprises cells. The fragmented extracellular matrix component in the extracellular-matrix-containing composition is an exogenous extracellular matrix component. At least a part of the cells may be adhering to the fragmented extracellular matrix component. The fragmented extracellular matrix component may be as described above.

The "three-dimensional tissue construct" refers to an assembly of cells in which the cells are three-dimensionally disposed via an extracellular matrix component such as a fibrillar collagen component and that is artificially produced through cell culture. The shape of the three-dimensional tissue construct is not limited to a particular shape, and examples thereof include a sheet, a sphere, an ellipsoid, and a cuboid. Here, biological tissues include blood vessels, sweat glands, lymphatic vessels, and sebaceous glands, and their configurations are more complex than that of the three-dimensional tissue construct. Therefore, the three-dimensional tissue construct and biological tissues can be easily distinguished from each other.

The cells are not limited to particular cells, and may be, for example, cells derived from an animal such as a human, a monkey, a dog, a cat, a rabbit, a pig, a bovine, a mouse, or a rat. The site from which the cells are derived is not limited to a particular site, and the cells may be somatic cells derived from, for example, the bone, muscle, internal organ, nerve, brain, bone, skin, or blood, and may be germ cells. Moreover, the cells may be induced pluripotent stem cells (iPS cells) or embryonic stem cells (ES cells), or cultured cells such as primary cultured cells, subcultured cells, and cell line cells. Specific examples of the cells include, but are not limited to, neurons, dendritic cells, immunocytes, vascular endothelial cells (e.g., human umbilical vein endothelial cells (HUVEC)), lymphatic endothelial cells, fibroblasts, colon cancer cells (e.g., human colon cancer cells (HT29)), carcinoma cells such as hepatic carcinoma cells, epithelial cells (e.g., human gingival epithelial cells), keratinocytes, cardiomyocytes (e.g., human-iPS-cell-derived cardiomyocytes (iPS-CM)), hepatocytes, pancreatic islet cells, tissue stem cells, and smooth muscle cells (e.g., aortic smooth muscle cells (Aorta-SMC)). One type of cells may be used singly, and multiple types of cells may be used in combination.

It is preferable that the cells include collagen-secreting cells, which secrete collagen such as fibrillar collagen. Examples of collagen-secreting cells include mesenchymal cells such as fibroblasts, chondrocytes, and osteoblasts, and fibroblasts are preferred. Examples of preferred fibroblasts include normal human dermal fibroblasts (NHDF), normal human cardiac fibroblasts (NHCF), and human gingival fibroblasts (HGF).

In the case that the three-dimensional tissue construct includes collagen-secreting cells as the cells, the three-dimensional tissue construct may contain endogenous collagen. The "endogenous collagen" refers to collagen which collagen-producing cells constituting the three-dimensional tissue construct produce. The endogenous collagen may be fibrillar collagen or non-fibrillar collagen.

In the case that the three-dimensional tissue construct includes collagen-secreting cells as the cells, the three-dimensional tissue construct may contain cells including collagen-secreting cells, a fragmented collagen component, and an endogenous collagen component. In this case, at least a part of the cells including collagen-secreting cells may be adhering to the fragmented extracellular matrix component and/or endogenous collagen component.

Conventional three-dimensional tissue constructs have low collagen concentration and high cell density. For this reason, conventional three-dimensional tissue constructs suffer from problems of contraction thereof due to tractive force by cells during or after culture, a tendency to be decomposed by an enzyme which cells produce during or after culture, and so forth. The three-dimensional tissue construct according to one embodiment have higher collagen concentration than conventional ones, and is less likely to undergo contraction and thus is stable.

The three-dimensional tissue construct may include collagen-secreting cells and cells other than collagen-secreting cells as the cells. Examples of cells other than collagen-producing cells include vascular endothelial cells (e.g., human umbilical vein endothelial cells (HUVEC)), cancer cells such as colon cancer cells (e.g., human colon cancer cells (HT29)) and hepatic cancer cells, cardiomyocytes (e.g., human-iPS-cell-derived cardiomyocytes (iPS-CM)), epithelial cells (e.g., human gingival epithelial cells), keratinocytes, lymphatic endothelial cells, neurons, hepatocytes, tissue stem cells, embryonic stem cells, induced pluripotent stem cells, adhesive cells (e.g., immunocytes), and smooth muscle cells (e.g., aortic smooth muscle cells (Aorta-SMC)). Preferably, the cells constituting the above three-dimensional tissue construct further include one or more types of cells selected from the group consisting of vascular endothelial cells, cancer cells, and cardiomyocytes.

The content ratio of collagen in the three-dimensional tissue construct may be 0.01 to 90% by mass based on the three-dimensional tissue construct (dry weight), and it is preferable that the content ratio of collagen in the three-dimensional tissue construct be 10 to 90% by mass, it is preferable that the content ratio of collagen in the three-dimensional tissue construct be 10 to 80% by mass, it is preferable that the content ratio of collagen in the three-dimensional tissue construct be 10 to 70% by mass, it is preferable that the content ratio of collagen in the three-dimensional tissue construct be 10 to 60% by mass, it is preferable that the content ratio of collagen in the three-dimensional tissue construct be 1 to 50% by mass, it is preferable that the content ratio of collagen in the three-dimensional tissue construct be 10 to 50% by mass, it is more preferable that the content ratio of collagen in the three-dimensional tissue construct be 10 to 30% by mass, and it is more preferable that the content ratio of collagen in the three-dimensional tissue construct be 20 to 30% by mass.

Here, the "collagen in the three-dimensional tissue construct" refers to collagen constituting the three-dimensional tissue construct, and may be endogenous collagen or collagen derived from the fragmented collagen component (exogenous collagen). It follows that in the case that the three-dimensional tissue construct contains endogenous collagen and exogenous collagen, the concentration of the above collagen constituting the three-dimensional tissue construct refers to the total concentration of endogenous collagen and exogenous collagen. The concentration of the above collagen can be calculated from the volume of the three-dimensional tissue construct obtained and the mass of the decellularized three-dimensional tissue construct.

Examples of methods for quantifying the amount of collagen in the three-dimensional tissue construct include a method of quantifying hydroxyproline as follows. Hydrochloric acid (HCl) is mixed in a lysis solution obtained by lysing the three-dimensional tissue construct; the resultant is incubated at a high temperature for a predetermined time; the temperature is then returned to room temperature; and centrifugation is performed and the resulting supernatant is diluted to a predetermined concentration to prepare a sample. Hydroxyproline standard solution is treated in the same manner as for the sample, and serial dilution is performed to prepare standards. The sample and standards are each subjected to a predetermined treatment with a hydroxyproline assay buffer and detection reagent, and absorbance at 570 nm is measured. The absorbance of the sample is compared with those of the standards to calculate the amount of collagen. Alternatively, a lysis solution obtained by directly suspending and dissolving the three-dimensional tissue construct in hydrochloric acid with a high concentration is centrifuged to collect the supernatant, which may be used for quantification of collagen. The three-dimensional tissue construct to be lysed may be in a state as recovered from culture solution, and may be subjected to dry treatment after recovery and lysed with the liquid components removed. If quantification of collagen is performed after the three-dimensional tissue construct in a state as recovered from culture solution is lysed, however, it is expected that culture medium components which the three-dimensional tissue construct has absorbed and a residual culture medium due to a problem in experimental operations cause variation of measurement values of the weight of the three-dimensional tissue construct, and hence it is preferred to use the weight after drying as a reference in order to stably measure the amount of collagen relative to the weight of the tissue or per unit weight.

More specific examples of methods for quantifying the amount of collagen include the following method.

(Preparation of Sample)

The whole of the three-dimensional tissue construct subjected to freeze-drying treatment is mixed with hydrochloric acid (6 mol/L HCl), the mixture is incubated in a heat block at 95°C for 20 hours or more, and the temperature is then returned to room temperature. Centrifugation is performed at 13000 g for 10 minutes, and the supernatant of the sample solution is then collected. The supernatant is appropriately diluted with hydrochloric acid (6 mol/L HCl) so that results of measurement described later can fall within the range of a calibration curve, and 200 $\mu$L of the resultant is diluted with 100 $\mu$L of ultrapure water to prepare a sample. The usage of the sample is 35 $\mu$L.

(Preparation of Standards)

Into a screwcap tube, 125 $\mu$L of standard solution (1200 $\mu$g/mL in acetic acid) and 125 $\mu$L of hydrochloric acid (12 mol/L HCl) are added and mixed together, the mixture is incubated in a heat block at 95°C for 20 hours, and the temperature is then returned to room temperature. Centrifugation is performed at 13000 g for 10 minutes, the supernatant is then diluted with ultrapure water to produce 300 $\mu$g/mL S1, and S1 is subjected to serial dilution to produce S2 (200 $\mu$g/mL), S3 (100 $\mu$g/mL), S4 (50 $\mu$g/mL), S5 (25 $\mu$g/mL), S6 (12.5 $\mu$g/mL), and S7 (6.25 $\mu$g/mL). Additionally, S8 (0 $\mu$g/mL), which consists only of 90 $\mu$L of hydrochloric acid (4mol/L HCl), is prepared.

(Assay)

The standards and the sample each in a volume of 35 $\mu$L are added to a plate (attached to a QuickZyme Total Collagen Assay Kit, QuickZyme Biosciences). To each well, 75 $\mu$L of assay buffer (attached to the kit) is added. The plate is sealed, and incubated at room temperature with shaking for 20 minutes. The plate is unsealed, and 75 $\mu$L of detection reagent (reagent A:B = 30 $\mu$L:45 $\mu$L, attached to the kit) is added to each well. The plate is sealed, and incubated at 60°C for 60 minutes while the solutions are mixed by shaking. The temperature is decreased to room temperature with ice, and the plate is unsealed and absorbance at 570 nm is measured. The absorbance of the sample is compared with those of the standards to calculate the amount of collagen.

Alternatively, collagen in the three-dimensional tissue construct may be specified with the area ratio or volume ratio. "Specifying with the area ratio or volume ratio" means that, for example, collagen in the three-dimensional tissue construct is made distinguishable from other tissue constituents by using a known staining method (e.g., immunostaining with an anti-collagen antibody, and Masson's trichrome staining) or the like, and then the ratio of regions in which collagen is present to the total of the three-dimensional tissue construct is calculated by using any of visual observation, microscopes, image analysis software, and so forth. In specifying with the area ratio, there is no limitation to which cross-section or surface in the three-dimensional tissue construct is used for specifying the area ratio, and in the case that the three-dimensional tissue construct is a sphere or the like, for example, a cross-sectional view along the generally central portion may be used for specification.

In specifying collagen in the three-dimensional tissue construct with the area ratio, the fraction of area is 0.01 to 99% based on the total area of the three-dimensional tissue construct, and it is preferable that the fraction of area be 1 to 99%, it is preferable that the fraction of area be 5 to 90%, it is preferable that the fraction of area be 7 to 90%, it is preferable that the fraction of area be 20 to 90%, and it is more preferable that the fraction of area be 50 to 90%. "Collagen in the three-dimensional tissue construct" is as described above. In the case that the three-dimensional tissue construct contains exogenous collagen derived from the fragmented collagen component, the fraction of area of collagen constituting the three-dimensional tissue construct refers to the fraction of combined areas of endogenous collagen and exogenous collagen. For example, as the three-dimensional tissue construct obtained is stained with Masson's trichrome, the fraction of area of collagen can be calculated as the fraction of area of collagen stained blue to the total area of a cross-section along a generally central portion of the three-dimensional tissue construct.

It is preferable for the three-dimensional tissue construct that the residue proportion after trypsin treatment with a trypsin concentration of 0.25% at a temperature of 37°C and pH 7.4 for a reaction time of 15 minutes be 70% or more, it is more preferable that the residue proportion be 80% or more, and it is even more preferable that the residue proportion be 90% or more. Such a three-dimensional tissue construct is less likely to undergo decomposition due to an enzyme during or after culture, and thus is stable. The residue proportion can be calculated, for example, from the mass of the three-dimensional tissue construct before and after trypsin treatment.

It is preferable for the three-dimensional tissue construct that the residue proportion after collagenase treatment with a collagenase concentration of 0.25% at a temperature of 37°C and pH 7.4 for a reaction time of 15 minutes be 70% or more, it is more preferable that the residue proportion be 80% or more, and it is even more preferable that the residue proportion be 90% or more. Such a three-dimensional tissue construct is less likely to undergo decomposition due to an enzyme during or after culture, and thus is stable.

It is preferable that the thickness of the three-dimensional tissue construct be 10 $\mu$m or more, it is more preferable that the thickness of the three-dimensional tissue construct be 100 $\mu$m or more, and it is even more preferable that the thickness of the three-dimensional tissue construct be 1000 $\mu$m or more. The structure of such a three-dimensional tissue construct is more similar to those of biological tissues, and preferred as an alternative for experimental animals and a material for transplantation. The upper limit of the thickness of the three-dimensional tissue construct is not limited to particular values, and may be, for example, 10 mm or less, 3 mm or less, 2 mm or less, 1.5 mm or less, or 1 mm or less.

Here, "the thickness of the three-dimensional tissue construct" refers to, in the case that the three-dimensional tissue construct is a sheet or cuboid, the distance between both ends in the direction perpendicular to a major surface. In the case that unevenness is present in the major surface, the thickness refers to the distance at the thinnest portion of the major surface.

In the case that the three-dimensional tissue construct is a sphere, the thickness refers to the diameter. Further, in the case that the three-dimensional tissue construct is an ellipsoid, the thickness refers to the minor axis. In the case that the three-dimensional tissue construct is a generally spherical or generally ellipsoidal shape and unevenness is present in the surface, the thickness refers to the shortest distance among those between two points at which a line passing through the center of gravity of the three-dimensional tissue construct and the surface intersect.

The three-dimensional tissue construct comprising a fragmented extracellular matrix component and cells can be produced, for example, with a method including: (1) a step of bringing an extracellular-matrix-containing composition containing a fragmented extracellular matrix component and an aqueous medium (first aqueous medium) into contact with cells (step (1)); and (2) a step of culturing the cells brought into contact with the extracellular-matrix-containing composition (step (2)).

In the method for producing a three-dimensional tissue construct, it is preferable that the cells be cells including collagen-producing cells. By using cells including collagen-secreting cells, a more stable three-dimensional tissue construct in which cells are homogeneously distributed can be obtained. Although details for the mechanism of providing such a three-dimensional tissue construct are unclear, the mechanism is inferred as follows.

In conventional methods of producing a three-dimensional tissue construct with use of a scaffold, it is difficult to distribute cells homogeneously into the inside of a scaffold because cells of interest are injected into a scaffold prepared in advance. In the case that the cells are cells including collagen-producing cells, the cells first come into contact with the surface of a fragmented extracellular matrix component and adhere to it. Thereafter, the cells by themselves produce a protein constituting an extracellular matrix component (e.g., collagen such as fibrillar collagen). The protein produced comes into contact with the surface of the fragmented extracellular matrix component and adheres to it to function as a crosslinking agent for the fragmented extracellular matrix component, and organization of the protein and so forth constituting the extracellular matrix component proceeds in an environment in which the cells are homogeneously present. As a result, a more stable three-dimensional tissue construct in which cells are homogeneously distributed is obtained. It should be understood, however, that the inference does not limit the present invention.

The production methods described in Patent Literatures 1 to 3 include many steps for producing a three-dimensional tissue construct, and require an operation time of about 1 hour. The production method according to the present embodiment enables production of a three-dimensional tissue construct in short operation time. Further, the production method according to the present embodiment enables production of a three-dimensional tissue construct in a simple manner. The production method described in Patent Literature 2 requires at least $10^6$ cells for producing a three-dimensional tissue construct having a thickness of about 1 mm. The production method according to the present embodiment enables production of a large-sized three-dimensional tissue construct having a thickness of 1 mm or more with a relatively small number of cells.

In step (1), an extracellular-matrix-containing composition containing a fragmented extracellular matrix component and a first aqueous medium is brought into contact with cells. Thereby, the fragmented extracellular matrix component in the extracellular-matrix-containing composition and the cells come into contact. The manner of bringing the extracellular-matrix-containing composition into contact with the cells is not limited to a particular method, and, for example, they may be brought into contact in a second aqueous medium. Examples of the manner of bringing into contact include a method of adding the extracellular-matrix-containing composition to a culture solution containing the cells, a method of adding the cells and, as necessary, a second aqueous medium to the extracellular-matrix-containing composition, and a method of adding the extracellular-matrix-containing composition and the cells to a second aqueous medium prepared in advance. The first and second aqueous media may be of the same type or different types.

In step (1), cells including collagen-producing cells and additional cells other than collagen-producing cells may be used. For the collagen-producing cells and the additional cells other than collagen-producing cells, the corresponding cells described above may be used. Through production of a three-dimensional tissue construct with use of collagen-producing cells and additional cells other than collagen-producing cells in combination, various model tissues can be produced. If NHCF and HUVEC are used, for example, a three-dimensional tissue construct including microvessels in the inside can be obtained. If NHCF and colon cancer cells are used, a model tissue of colon cancer can be obtained. If NHCF and iPS-CM are used, a model tissue of myocardia that exhibit synchronized beating can be obtained.

The concentration of the fragmented extracellular matrix component in step (1) may be appropriately determined according to the intended shape and thickness of the three-dimensional tissue construct, the size of an incubator, and so forth. For example, the concentration of the fragmented extracellular matrix component may be 0.1 to 90% by mass or 1 to 30% by mass based on the total amount of the extracellular-matrix-containing composition.

The quantity of the fragmented extracellular matrix component in step (1) may be 0.1 to 100 mg or 1 to 50 mg per $1 \times 10^5$ cells.

It is preferable that the mass ratio of the fragmented extracellular matrix component to the cells (extracellular matrix component/cells) in step (1) be 1/1 to 1000/1, it is more preferable that the mass ratio be 9/1 to 900/1, and it is even more preferable that the mass ratio be 10/1 to 500/1.

In the case that collagen-producing cells and additional cells are used in combination, the number ratio of the collagen-producing cells in step (1) to the additional cells (ratio of collagen-producing cells/additional cells in step (1)) may be 9/1 to 99/1, 50/50 to 80/20, 20/80 to 50/50, or 10/90 to 50/50.

A step of precipitating both the fragmented extracellular matrix component and the cells in the aqueous medium may be further included between step (1) and step (2). By performing such a step, the distribution of the fragmented extracellular matrix component and the cells in the three-dimensional tissue construct becomes more homogeneous. Specific examples of such methods include, but are not limited to, a method of centrifuging a culture solution containing the fragmented extracellular matrix component and the cells.

Step (1) may be performed by forming a layer of cells in an aqueous medium (second aqueous medium), followed by bringing an extracellular-matrix-containing composition containing a fragmented extracellular matrix component and an aqueous medium (first aqueous medium) into contact with the layer. By forming a layer of cells before bringing into contact with an extracellular-matrix-containing composition, a three-dimensional tissue construct whose lower part has a high cell density can be produced. By forming a layer of cells including collagen-producing cells before bringing into contact with an extracellular-matrix-containing composition, a three-dimensional tissue construct whose lower part has a high cell density of cells including collagen-producing cells can be produced. For some types of cells to be used (e.g., aortic smooth muscle cells), a tissue more similar to the corresponding tissue in a living body can be produced through that method.

In step (2), the cells brought into contact with the extracellular-matrix-containing composition are cultured. Thereby, a three-dimensional tissue construct is formed. After step (2), a step of further bringing into contact with cells and culturing the cells may be included as step (3). These cells may be of the same type as the cells used in step (1), or of different type. In the case that cells to be used in step (1) include cells other than collagen-producing cells, for example, cells to be used in step (3) may include collagen-producing cells. In the case that cells to be used in step (1) include collagen-producing cells, for example, cells to be used in step (3) may include cells other than collagen-producing cells. Both of cells to be used in step (1) and cells to be used in step (3) may include collagen-producing cells, and both of cells to be used in step (1) and cells to be used in step (3) may include cells other than collagen-producing cells. Through step (3), a three-dimensional tissue construct of bilayer structure can be produced. In the case that aortic smooth muscle cells and vascular endothelial cells are used, and in the case that human-skin-derived fibroblasts and human epidermal keratinocytes are used, for example, a tissue more similar to the corresponding tissue in a living body can be produced through that method. In the case that human gingival fibroblasts and gingival epithelial cells are used, for example, a three-dimensional tissue construct of bilayer structure without tissue contraction and tissue cracking can be produced through that method.

The manner of culturing cells is not limited to a particular method, and a preferred culture method may be used for culturing according to the type of cells to be cultured. For example, the culture temperature may be 20°C to 40°C or 30°C to 37°C. The pH of the culture medium may be 6 to 8 or 7.2 to 7.4. The culture period may be 1 day to 2 weeks or 1 week to 2 weeks.

The culture medium is not limited to a particular culture medium, and a preferred culture medium may be selected according to the type of cells to be cultured. Examples of such culture media include an Eagle's MEM, a DMEM, a Modified Eagle' Medium (MEM), Minimum Essential Medium, an RPMI, and a GlutaMax Medium. The culture medium may be a medium with serum, or a serum-free medium. Further, the liquid culture medium may be a mixed culture medium obtained by mixing two or more culture media.

The cell density in the culture medium in step (2) may be appropriately determined according to the intended shape and thickness of the three-dimensional tissue construct, the size of an incubator, and so forth. For example, the cell density in the culture medium in step (2) may be 1 to $10^8$ cells/mL or $10^3$ to $10^7$ cells/mL. The cell density in the culture medium in step (2) may be the same as the cell density in the aqueous medium in step (1).

It is preferable that the contraction rate of the three-dimensional tissue construct during culture be 20% or less, it is more preferable that the contraction rate be 15% or less, and it is even more preferable that the contraction rate be 10% or less. The contraction rate can be calculated, for example, by using the following expression, wherein L1 denotes the length of the longest part of the three-dimensional tissue construct 1 day after culture, and L3 denotes the length of the corresponding part of the three-dimensional tissue construct 3 days after culture.

$$\text{Contraction rate } (\%) = \{(L1\text{-}L3)/L1\} \times 100$$

Through the above-described production method, for example, a three-dimensional tissue construct comprising cells and an extracellular matrix component, wherein the content ratio of collagen is 10% by mass to 90% by mass based on the three-dimensional tissue construct, can be produced.

The method for producing a three-dimensional tissue construct may include (4) a step of cooling the three-dimensional tissue construct formed to allow the fragmented extracellular matrix component to undergo solation (step (4)), and (5) a step of removing after step (4) the fragmented extracellular matrix component after undergoing solation. Cooling of the three-dimensional tissue construct may be performed in an aqueous medium.

Through inclusion of step (4) and step (5) in the method for producing a three-dimensional tissue construct, at least a part of extracellular matrix derived from the fragmented extracellular matrix component (exogenous extracellular matrix) can be removed after formation of the three-dimensional tissue construct.

In step (4), the three-dimensional tissue construct is cooled to allow the fragmented extracellular matrix component to undergo solation. Cooling of the three-dimensional tissue construct may be performed in an aqueous medium. Cooling of the three-dimensional tissue construct may be performed by decreasing the temperature of a culture solution containing the three-dimensional tissue construct and an aqueous medium. The temperature in cooling the three-dimensional tissue construct (cooling temperature) may be appropriately set according to the type of the aqueous medium and so forth. The cooling temperature may be, for example, 15°C or less, or 4°C, or over 0°C.

In step (5), the fragmented extracellular matrix component after undergoing solation (in a sol state) is removed. Thereby, at least a part or all of the fragmented extracellular matrix component, which is an exogenous extracellular matrix component, can be removed from the three-dimensional tissue construct.

Removal of the fragmented extracellular matrix component in a sol state can be performed, for example, by suspending the three-dimensional tissue construct in which a part or all of the fragmented extracellular matrix component is in a sol state in an aqueous medium (e.g., phosphate-buffered saline), and centrifuging the suspension to remove the supernatant.

After culturing the three-dimensional tissue construct, which is formed with an extracellular-matrix-containing composition that exhibits thermally reversible sol-gel transition, for a certain period of time under normal physiological conditions (pH 7 to 8, 37°C), gelation has proceeded to some degree with cells included in the aqueous medium. By cooling the three-dimensional tissue construct in an aqueous medium, the fragmented extracellular matrix component, which is an exogenous extracellular matrix component, can be allowed to undergo solation and removed. Through removal of the exogenous extracellular matrix component among the constituent components of the three-dimensional tissue construct, a more in-vivo-like tissue without any exogenous component can be obtained.

When the extracellular-matrix-containing composition, which contains the fragmented extracellular matrix component and an aqueous medium, is cooled, the fragmented extracellular matrix component becomes in a sol state. Hence, cells can be recovered from the three-dimensional tissue construct with ease, for example, by allowing the fragmented extracellular matrix component to undergo solation after the three-dimensional tissue construct is formed by using the above-described production method, and removing the fragmented extracellular matrix component in a sol state. Accordingly, the present invention provides, in one aspect, a method for recovering a cell from a three-dimensional tissue construct comprising a fragmented extracellular matrix component and a cell, comprising: a step of cooling the three-dimensional tissue construct formed to allow the fragmented extracellular matrix component to undergo solation; and a step of removing the fragmented extracellular matrix component after undergoing solation.

In removing the fragmented extracellular matrix component for the purpose of recovering cells from the three-dimensional tissue construct, removal of cells can be more efficiently performed by removing exogenous components followed by suspending the tissue in a solution containing collagenase in a concentration range that causes no significantly large influence on the survival rate of cells.

**Examples**

Hereinafter, the present invention will be specifically described on the basis of Examples; however, the present invention is not limited to them.

(Example 1)

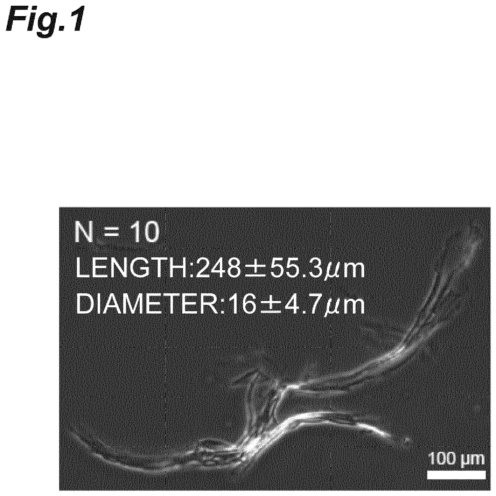

Fifty milligrams of a freeze-dried product of pepsin-treated porcine skin collagen type I (produced by NH Foods Ltd.) was dispersed in 5.0 mL of $10\times$ phosphate-buffered saline (PBS, pH 7.4), and this dispersion was homogenized by using a homogenizer (VIOLAMO) for 6 minutes to obtain a suspension of a fragmented collagen component (CMF). Washing and centrifugation were performed for the suspension of the fragmented collagen component. Figure 1 shows a microphotograph of the fragmented collagen component obtained. The fragmented collagen component had an average diameter of 16 $\mu$m $\pm$ 4.7 $\mu$m and an average length (length) of 248 $\mu$m $\pm$ 55.3 $\mu$m (number of samples: 10).

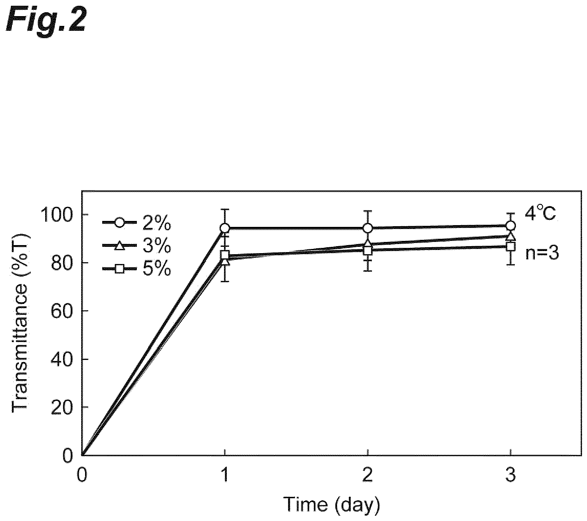

The fragmented collagen component obtained was diluted with $1\times$ phosphate-buffered saline to prepare a solution containing 2, 3, or 5% by mass of the fragmented collagen component (fragmented-collagen-containing solutions) in each volume of 5.0 mL. The fragmented-collagen-containing solutions were stored at 4°C for 3 days, and the temporal transition of transmittance (%T) of each fragmented-collagen-containing solution during this period was observed on each day. Figure 2 shows the results. As demonstrated in Figure 2, for all the concentrations the fragmented-collagen-containing solution had sufficient transmittance and was confirmed to have undergone solation during storage at 4°C. The average values of transmittance from the initiation of storage at 4°C to the time point after the lapse of 3 days for the 2, 3, and 5% by mass fragmented-collagen-containing solutions were 94.9%, 90.4%, and 86.1%, respectively. Measurement of transmittance was performed by using a V-670 spectrophotometer (JASCO Corporation) at a wavelength of 500 nm.

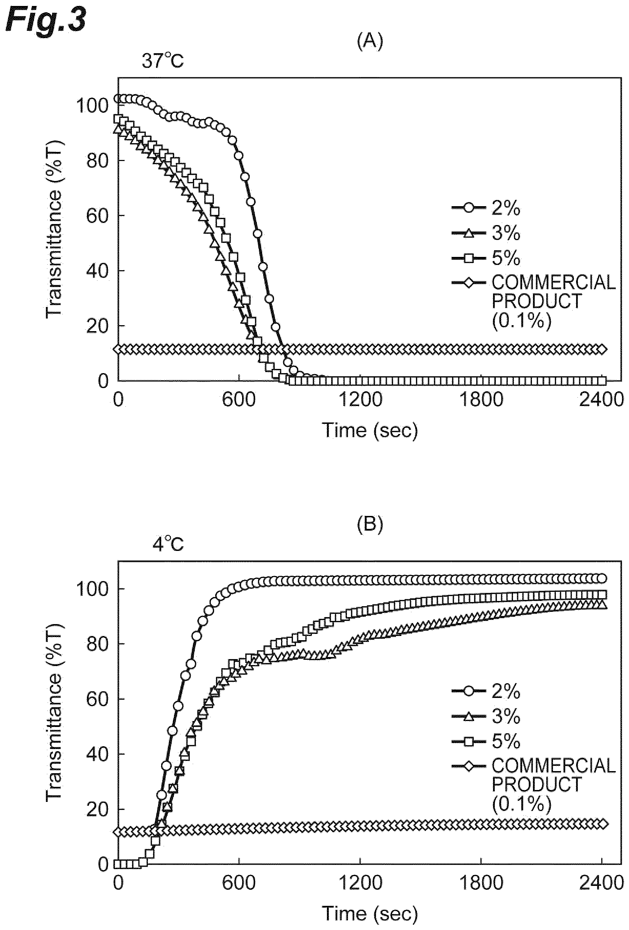

The 2, 3, or 5% by mass fragmented-collagen-containing solutions and a commercially available collagen solution (0.1% by mass, produced by Nippi, Incorporated) were repeatedly subjected to temperature change such that the temperature was switched between 37°C and 4°C every 2400 seconds and retained, and the variation of transmittance for light with a wavelength of 500 nm was checked. Figures 3A and 3B show the results. The results found that the 2, 3, and 5% by mass fragmented-collagen-containing solutions all exhibited reversible thermal response. Specifically, the fragmented-collagen-containing solutions were each in a gel state at 37°C and in a sol state at 4°C. By contrast, the commercially available collagen solution did not exhibit such thermal response, and remained in a gel state.

When the temperature of each of the 2, 3, and 5% by mass fragmented-collagen-containing solutions was increased at 1°C/min from 4°C to 40°C, they underwent transition from a sol state into a gel state at 30°C, 32°C, and 30°C (gel transition temperature), respectively. When the temperature of each of the 2, 3, and 5% by mass fragmented-collagen-containing solutions was decreased at 1°C/min from 40°C to 4°C, they underwent transition from a gel state into a sol state at 10°C, 13°C, and 15°C (sol transition temperature), respectively.

The commercially available collagen solution was one prepared in accordance with a protocol provided by Nippi, Incorporated. The collagen solution was prepared by adding $5\times$ D-MEM (600 $\mu$L), FBS (300 $\mu$L), and sterile water (1100 $\mu$L) to 0.3% by mass acetic acid solution (1000 $\mu$L) of type I bovine skin (acid-soluble) produced by Nippi, Incorporated for 3-fold dilution.

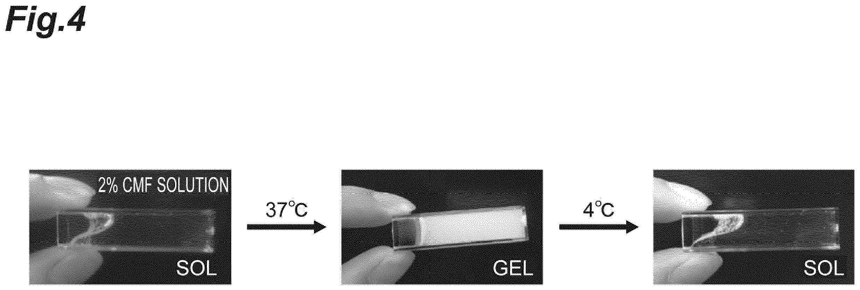

Figure 4 shows results of visual observation for thermal response when the 2% by mass fragmented-collagen-containing solution (2% CMF solution) was used. As demonstrated, the fragmented-collagen-containing solution underwent gelation at 37°C, and significant lowering of transmittance was confirmed. It was confirmed that the fragmented-collagen-containing solution again underwent solation during storage at 4°C, and the transmittance increased.

Fifty milligrams of a freeze-dried product of pepsin-treated porcine skin collagen type I (produced by NH Foods Ltd.) was dispersed in 5.0 mL of 70% by volume ethanol aqueous solution, and this dispersion was homogenized by using a homogenizer for 2 minutes, and then diluted with $1\times$ phosphate-buffered saline to obtain a fragmented-collagen-containing solution containing a fragmented collagen component (CMF) and phosphate-buffered saline. Fragmented collagen was successfully produced also through preparation involving dispersing in advance in 70% by volume ethanol aqueous solution.

(Example 2)

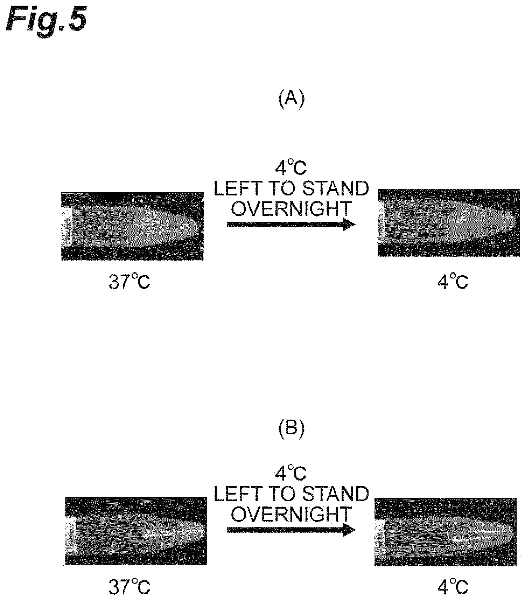

Examined was whether the fragmented-collagen-containing solution used in Example 1 would exert thermal response on undergoing gelation under the same conditions as for the commercially available collagen solution. Specifically, 0.1% by mass of the fragmented collagen component was suspended in a D-MEM solution containing 0.5 mM acetic acid and 10% FBS, and NaOH was added thereto to adjust the pH to 7.3. The resultant was directly allowed to gelation at 37°C. Thereafter, the fragmented-collagen-containing solution was stored at 4°C for a day, and whether it would undergo solation was checked. The result found that the commercially available collagen solution retained the gel state once it underwent gelation even when being stored at 4°C (see Figure 5A). By contrast, the fragmented-collagen-containing solution was found to undergo solation through storage at 4°C (see Figure 5B).

(Example 3: Production of Three-dimensional tissue construct Using Normal Human Dermal Fibroblasts (NHDF))

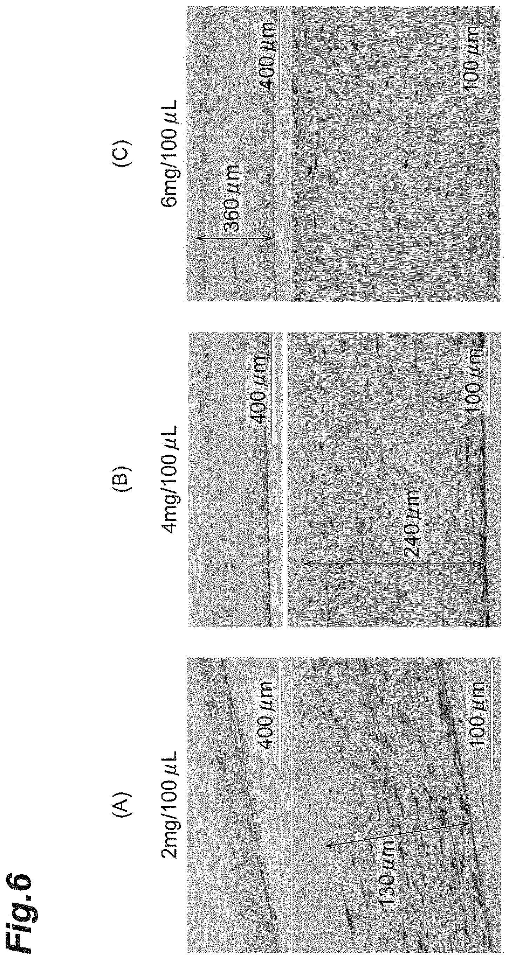

A freeze-dried product of porcine skin collagen type I produced by NH Foods Ltd. was suspended in 10× phosphate-buffered saline (X10 PBS), and homogenized by using a homogenizer for 6 minutes to obtain a fragmented collagen component. The fragmented collagen component was dispersed in a medium (DMEM) containing serum so that the content reached 20 mg/mL, 40 mg/mL, or 60 mg/mL based on the total amount of the dispersion (extracellular-matrix-containing composition). In 100 $\mu$L of each dispersion obtained (respectively containing approximately 2 mg, 4 mg, or 6 mg of the fragmented collagen component), $5.0 \times 10^5$ cells of normal human dermal fibroblasts (NHDF) were suspended, and added to a 24-well cell culture insert (produced by Corning Incorporated) and cultured for 5 days. Thereafter, the three-dimensional tissue constructs obtained were stained with hematoxylin-eosin (HE). Figure 6 shows the results. As demonstrated in Figure 6, tissue formation with a thickness corresponding to collagen concentration was found for the tissues formed through suspension in each fragmented collagen dispersion. In addition, agglomeration on the insert was not found.

(Example 4: Removal of Fragmented Collagen Component and Recovery of Cells from Three-dimensional tissue construct)

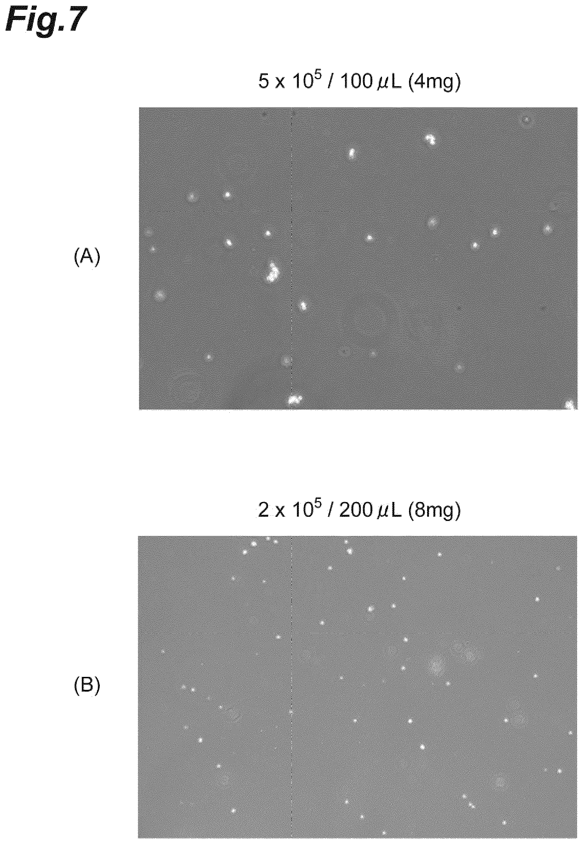

A freeze-dried product of porcine skin collagen type I produced by NH Foods Ltd. was suspended in 10× phosphate-buffered saline (X10 PBS), and homogenized by using a homogenizer for 6 minutes to obtain a fragmented collagen component. The fragmented collagen component was dispersed in a medium (DMEM) containing serum so that the content reached 40 mg/mL based on the total amount of the dispersion (extracellular-matrix-containing composition). In 100 $\mu$L and 200 $\mu$L of the dispersion obtained (respectively containing approximately 4 mg and 8 mg of the fragmented collagen component), $5.0 \times 10^5$ cells and $2.0 \times 10^5$ cells of normal human dermal fibroblasts (NHDF) were respectively suspended, and each added to a 24-well cell culture insert and cultured for 5 days. Thereafter, the suspensions were left to stand at 4°C or less with ice-cooling, and each tissue was suspended in 200 $\mu$L of PBS and centrifuged (3500 rpm, 1 minute) to remove the supernatant. Further, 1 mL of DMEM was added to each precipitated component, 300 $\mu$L of 1 mg/mL collagenase solution was added thereto, and cell counting was then performed to calculate viable cell ratios and recovery rates from initial amounts of seeding. Figure 7 shows photographs showing cells after recovery. It was found that the viable cell ratios in the fractions recovered in this Example were over 75% (case with 4 mg of fragmented collagen component: 75%, case with 8 mg of fragmented collagen component: 88%), and about 30 to 40% of viable cells as calculated from the initial amounts of seeding were successfully recovered (case with 4 mg of fragmented collagen component: 31%, case with 8 mg of fragmented collagen component: 43%). The collagenase solution added was for the purpose of performing more accurate cell-counting.

(Example 5: Variation of Transition Temperature with Fragmented Collagen Concentration)

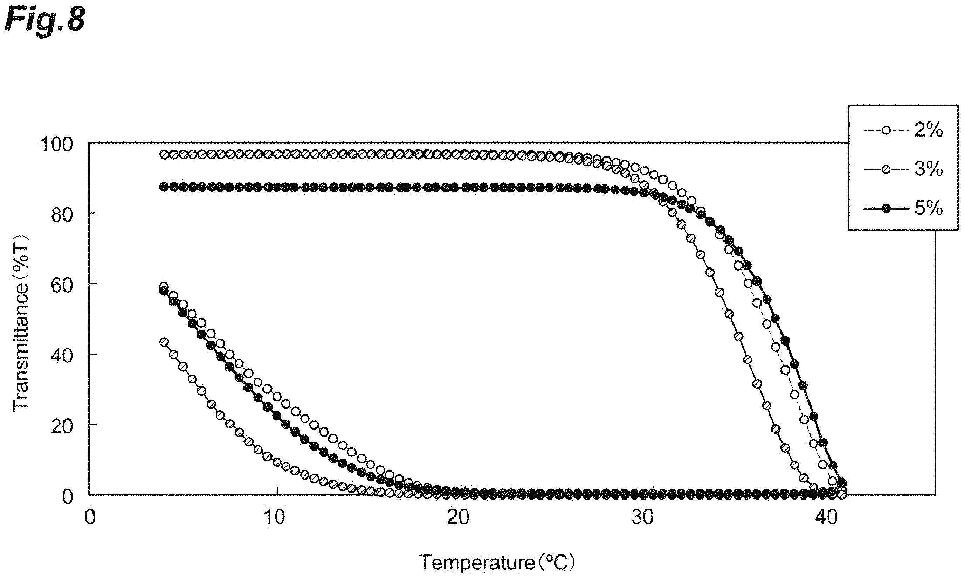

Fragmented-collagen-containing solutions with concentrations of 2% by mass, 3% by mass, and 5% by mass were prepared. Each concentration is that of the fragmented collagen component based on the total mass of the aqueous solution. For the fragmented-collagen-containing solutions with different concentrations, transmittance at a wavelength of 500 nm when the temperature was changed from 4°C to 40°C or from 40°C to 4°C was measured. The temperature was increased or decreased at 0.5°C/min.
As demonstrated in Figure 8, the temperature at solation from gel was 34 to 37°C, and the temperature at solation from gel was 3 to 6°C. The gelation temperature and solation temperature of the fragmented collagen component were 35.5°C ± 2°C and 4.5°C ± 2°C, respectively.

(Example 6: Effect of Origin of Collagen)

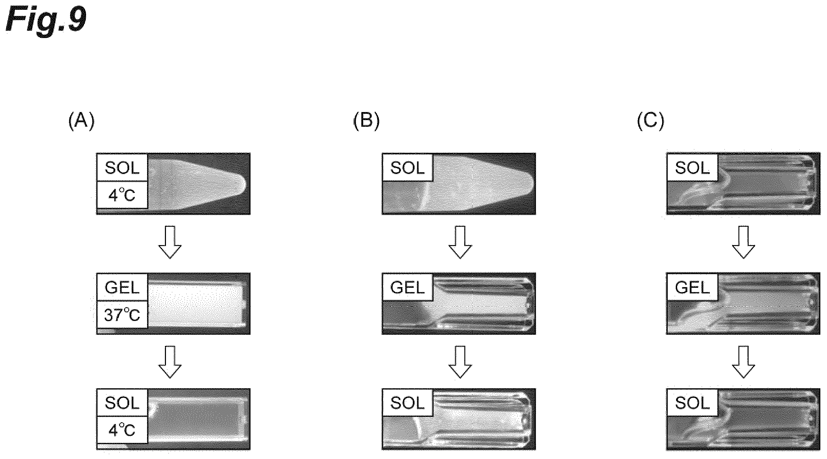

By using pig-derived, bovine-derived, and human-derived skin collagens (type I), fragmented-collagen-containing solutions with a concentration of 2% by mass were prepared. The concentration is that of each fragmented collagen component based on the total mass of the aqueous solution. The temperature of each fragmented-collagen-containing solution in a sol state was increased to 37°C to allow it to undergo gelation. After gelation, each fragmented-collagen-containing solution was cooled to 4°C to allow it to undergo solation. It was found that thermally reversible sol-gel transition occurred for any fragmented collagen component of animal origin, namely, any of the pig-derived, bovine-derived, and human-derived fragmented collagen components. In Figure 9, (A), (B), and (C) respectively show thermally reversible sol-gel transition of the pig-derived, bovine-derived, and human-derived fragmented-collagen-containing solutions.
The pig-derived and human-derived fragmented collagen components were superior in solubility to the bovine-derived fragmented collagen component, and the pig-derived fragmented collagen component was particularly significantly superior in solubility. The thermoresponsivity of the pig-derived fragmented collagen was superior to those of the bovine-derived and human-derived fragmented collagens.

(Example 7: Confirmation of Triple Helix Structure with CD Spectrum)

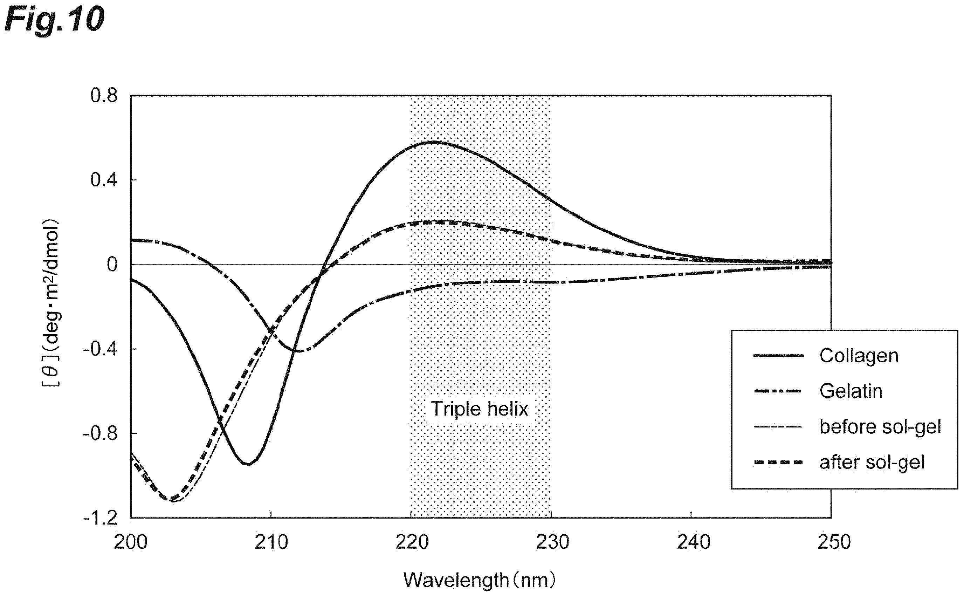

The fragmented collagen component was analyzed with the CD spectrum. Figure 10 shows the results. The CD spectrum measurement was performed by using a circular dichroism spectrometer (JASCO Corporation, J-725) in accordance with a procedure recommended by the manufacturer. The fragmented collagen component with a concentration of 0.05 mg/mL (final concentration) was dissolved in 50 mM acetic acid, and subjected to measurement.
As demonstrated in Figure 10, it was found that a peak near 220 nm, which indicates a triple helix structure, was present for the fragmented collagen component. Because no variation of waveform caused by the sol-gel transition was found, it was confirmed that the sol-gel transition did not affect the structure of the fragmented collagen component. The fragmented collagen component is inferred to have high sol-gel transition properties because the fragmented collagen component retains the triple helix structure.

(Example 8: Confirmation of Molecular Weight of Fragmented Collagen Component with SDS-PAGE)

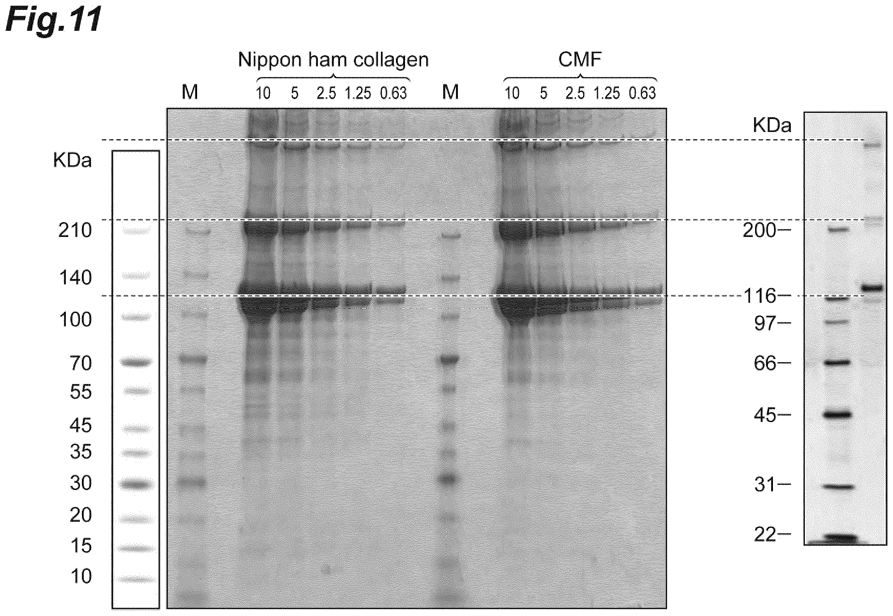

Molecular weight was compared between the fragmented collagen component and the collagen component (non-fragmented collagen component) with SDS-PAGE. Electrophoresis was performed by using a gradient gel with an acrylamide concentration of 4 to 20% with addition of 2.15% sodium dodecylsulfate (SDS) to electrophoresis samples of the fragmented collagen component. It was found that there was no difference between the molecular weight of the fragmented collagen component per molecule and that of the collagen component (Figure 11). It was confirmed that the fragmented collagen component obtained by using the method described in Example 1 became smaller not because the molecular structure was broken, but because the collagen component was fibrillated.

**Claims**

1. An extracellular-matrix-containing composition comprising:

   a fragmented extracellular matrix component; and
   an aqueous medium.

2. The extracellular-matrix-containing composition according to claim 1, wherein the fragmented extracellular matrix component comprises a fragmented collagen component.

3. The extracellular-matrix-containing composition according to claim 1 or 2, wherein at least a part of the fragmented extracellular matrix component is fibrillar.

4. The extracellular-matrix-containing composition according to any one of claims 1 to 3, wherein an average length of the fragmented extracellular matrix component is 100 nm to 400 μm.

5. The extracellular-matrix-containing composition according to any one of claims 1 to 4, wherein a content of the extracellular matrix component is 1 mg/mL or more and 100 mg/mL or less based on a total amount of the extracellular-matrix-containing composition.

6. The extracellular-matrix-containing composition according to any one of claims 1 to 5, wherein the fragmented extracellular matrix component is naturally-occurring.

7. The extracellular-matrix-containing composition according to any one of claims 1 to 6, wherein the extracellular-matrix-containing composition undergoes gelation from a sol state at 35.5°C ± 2°C, and undergoes solation from a gel state at 4.5°C ± 2°C.

8. A temporary scaffold for three-dimensional tissue construct formation, the temporary scaffold comprising:
the extracellular-matrix-containing composition according to any one of claims 1 to 7.

9. A three-dimensional tissue construct formation agent with a high content ratio of an extracellular matrix component, the three-dimensional tissue construct formation agent comprising:
the extracellular-matrix-containing composition according to any one of claims 1 to 7.

10. A three-dimensional tissue construct comprising:
the extracellular-matrix-containing composition according to any one of claims 1 to 7.

11. A method for recovering a cell from a three-dimensional tissue construct comprising a fragmented extracellular matrix component and a cell, comprising:

a step of cooling the three-dimensional tissue construct to allow the fragmented extracellular matrix component to undergo solation; and
a step of removing the fragmented extracellular matrix component after undergoing solation.

Fig.1

*Fig.2*

# Fig.3

(A)

(B)

Fig.4

# Fig.5

(A)

4°C
LEFT TO STAND
OVERNIGHT

37°C

4°C

(B)

4°C
LEFT TO STAND
OVERNIGHT

37°C

4°C

# Fig.6

(A)

2mg/100 μL

(B)

4mg/100 μL

(C)

6mg/100 μL

*Fig.7*

$5 \times 10^5 / 100 \, \mu L$ (4mg)

(A)

$2 \times 10^5 / 200 \, \mu L$ (8mg)

(B)

Fig.8

EP 3 786 175 A1

Fig.9

# Fig.10

EP 3 786 175 A1

# Fig.11

**INTERNATIONAL SEARCH REPORT**

| International application No. |
| --- |
| PCT/JP2019/018271 |

A. CLASSIFICATION OF SUBJECT MATTER
Int.Cl. C07K14/47(2006.01)i, C12N5/071(2010.01)i

According to International Patent Classification (IPC) or to both national classification and IPC

B. FIELDS SEARCHED

Minimum documentation searched (classification system followed by classification symbols)
Int.Cl. C07K14/47, C12N5/071

Documentation searched other than minimum documentation to the extent that such documents are included in the fields searched

| | |
| --- | --- |
| Published examined utility model applications of Japan | 1922–1996 |
| Published unexamined utility model applications of Japan | 1971–2019 |
| Registered utility model specifications of Japan | 1996–2019 |
| Published registered utility model applications of Japan | 1994–2019 |

Electronic data base consulted during the international search (name of data base and, where practicable, search terms used)
JSTPlus/JMEDPlus/JST7580 (JDreamIII), CAplus/MEDLINE/EMBASE/BIOSIS/WPIDS (STN)

C. DOCUMENTS CONSIDERED TO BE RELEVANT

| Category* | Citation of document, with indication, where appropriate, of the relevant passages | Relevant to claim No. |
| --- | --- | --- |
| X<br>Y<br>A | 加藤菜津子他, コラーゲンマイクロファイバーを用いた三次元がん－間質組織体の構築, 第66回高分子学会年次大会 高分子学会予稿集, 2017, vol. 66, no. 1, 2Pb096, entire text, non-official translation (KATO, Natsuko et al., "Construction of three-dimensional cancer-interstitial tissue using collagen microfiber", Preprints of the 66th SPSJ Annual Meeting) | 1-10<br>8-10<br>11 |
| X<br>Y<br>A | 西宏基他, コラーゲンマイクロファイバーを用いた iPS 細胞由来心筋細胞組織の構築, 第66回高分子学会年次大会 高分子学会予稿集, 2017, vol. 66, no. 1, 2Pa097, entire text, non-official translation (NISHI, Koki et al., "Construction of iPS cell-derived cardiomyocyte tissue using collagen microfiber", Preprints of the 66th SPSJ Annual Meeting) | 1-10<br>8-10<br>11 |

☒ Further documents are listed in the continuation of Box C.     ☐ See patent family annex.

| | |
| --- | --- |
| * Special categories of cited documents:<br>"A" document defining the general state of the art which is not considered to be of particular relevance<br>"E" earlier application or patent but published on or after the international filing date<br>"L" document which may throw doubts on priority claim(s) or which is cited to establish the publication date of another citation or other special reason (as specified)<br>"O" document referring to an oral disclosure, use, exhibition or other means<br>"P" document published prior to the international filing date but later than the priority date claimed | "T" later document published after the international filing date or priority date and not in conflict with the application but cited to understand the principle or theory underlying the invention<br>"X" document of particular relevance; the claimed invention cannot be considered novel or cannot be considered to involve an inventive step when the document is taken alone<br>"Y" document of particular relevance; the claimed invention cannot be considered to involve an inventive step when the document is combined with one or more other such documents, such combination being obvious to a person skilled in the art<br>"&" document member of the same patent family |

| Date of the actual completion of the international search<br>29 July 2019 (29.07.2019) | Date of mailing of the international search report<br>13 August 2019 (13.08.2019) |
| --- | --- |
| Name and mailing address of the ISA/<br>Japan Patent Office<br>3-4-3, Kasumigaseki, Chiyoda-ku,<br>Tokyo 100-8915, Japan | Authorized officer<br><br>Telephone No. |

Form PCT/ISA/210 (second sheet) (January 2015)

## INTERNATIONAL SEARCH REPORT

| International application No. |
|---|
| PCT/JP2019/018271 |

C (Continuation). DOCUMENTS CONSIDERED TO BE RELEVANT

| Category* | Citation of document, with indication, where appropriate, of the relevant passages | Relevant to claim No. |
|---|---|---|
| X<br>Y<br>A | 米田美咲他，コラーゲンマイクロファイバーによる高濃度細胞外マトリックスと毛細血管網を有する三次元間質組織体の構築，第66回高分子学会年次大会 高分子学会予稿集，2017, vol. 66, no. 2, 2Q10, entire text, non-official translation (YONEDA, Misaki et al., "Construction of three-dimensional interstitial tissue with high concentration extracellular matrix and capillary network by collagen microfiber", Preprints of the 66th SPSJ Annual Meeting) | 1-10<br>8-10<br>11 |
| X<br>Y<br>A | 加藤菜津子他，コラーゲンマイクロファイバーを用いた高密度ECMを有する三次元ヒトがん－間質組織体の構築，第66回高分子学会年次大会 高分子学会予稿集，2017, vol. 66, no. 2, 2Q11, entire text, non-official translation (KATO, Natsuko et al., "Construction of 3D human cancer interstitial tissue with high density ECM using collagen microfiber", Preprints of the 66th SPSJ Annual Meeting) | 1-10<br>8-10<br>11 |
| X<br>Y<br>A | 松崎典弥他，コラーゲンマイクロファイバーを用いた沈殿培養による高密度ECMを有する3D－結合組織の構築，第66回高分子学会年次大会 高分子学会予稿集，2017, vol. 66, no. 2, 3N02, entire text, non-official translation (MATSUSAKI, Michiya et al., "Construction of 3D-connective tissue having high density ECM through sedimentation culture in which collagen microfiber is used", Preprints of the 66th SPSJ Annual Meeting) | 1-10<br>8-10<br>11 |
| X<br>Y<br>A | 西宏基他，コラーゲンマイクロファイバーを用いたiPS細胞由来心筋細胞線維化モデルの構築，第66回高分子学会年次大会 高分子学会予稿集，2017, vol. 66, no. 2, 3N03, entire text, non-official translation (NISHI, Koki et al., "Establishment of iPS cell-derived cardiac fibrosis model using collagen microfiber", Preprints of the 66th SPSJ Annual Meeting) | 1-10<br>8-10<br>11 |
| X<br>Y<br>A | 松崎典弥他，生体類似の高密度な細胞外マトリックスと毛細血管を有する三次元間質組織体の構築，日本化学会第97春季年会(2017)講演予稿集，2017, 3C1-03, entire text, non-official translation (MATSUSAKI, Michiya et al., "Construction of three-dimensional stromal tissue with biosimilar dense extracellular matrix and capillaries", Lecture preprints of the 97th spring annual conference of the Chemical Society of Japan (2017)) | 1-10<br>8-10<br>11 |

Form PCT/ISA/210 (continuation of second sheet) (January 2015)

**EP 3 786 175 A1**

**INTERNATIONAL SEARCH REPORT**

International application No.

PCT/JP2019/018271

C (Continuation).   DOCUMENTS CONSIDERED TO BE RELEVANT

| Category* | Citation of document, with indication, where appropriate, of the relevant passages | Relevant to claim No. |
|---|---|---|
| X<br>Y<br>A | 加藤菜津子他，コラーゲンマイクロファイバーを用いた高密度ECMを有する三次元ヒトがん－間質組織体の構築，第17回日本再生医療学会総会, March 2018, 0-11-6, entire text, non-official translation (KATO, Natsuko et al., "Construction of 3D human cancer-interstitial tissue with high-density ECM using collagen microfiber", The 17th Congress of the Japanese Society for Regenerative Medicine) | 1-10<br>8-10<br>11 |
| X<br>Y<br>A | 西宏基他，コラーゲンマイクロファイバーとiPS細胞由来心筋細胞によるヒト心筋線維化モデルの構築，第17回日本再生医療学会総会, March 2018, 0-31-4, entire text, non-official translation (NISHI, Koki et al., "Construction of human myocardial fibrosis model using collagen microfiber and iPS cell-derived cardiomyocytes", The 17th Congress of the Japanese Society for Regenerative Medicine) | 1-10<br>8-10<br>11 |
| X<br>Y<br>A | JP 49-121859 A (HYOGO PREFECTURE) 21 November 1974, claims, examples, p. 1, lower right column, bottom paragraph to p. 3, upper right column, paragraph [0001] (Family: none) | 1-3,5-7<br>8-10<br>4,11 |
| P,X | 湯川優一他，感熱応答性を有するコラーゲンゲルの調製，第67回高分子学会年次大会 高分子学会予稿集, 08 May 2018, vol. 67, no. 1, 2H19, entire text, non-official translation (YUKAWA, Yuichi et al., "Preparation of collagen gel with thermo-responsiveness", Preprints of the 67th SPSJ Annual Meeting) | 1-10 |
| P,X | 湯川優一他，高濃度化で発現した哺乳類コラーゲンの感熱応答挙動の解析，第40回日本バイオマテリアル学会大会予稿集, November 2018, p. 310, 1P-055, entire text, non-official translation (YUKAWA, Yuichi et al., "Analysis of thermo-responsive behavior of mammalian collagen expressed at high concentration", Preprints of the 40th conference of Japanese Society for Biomaterials) | 1-10 |

Form PCT/ISA/210 (continuation of second sheet) (January 2015)

30

## REFERENCES CITED IN THE DESCRIPTION

**Patent documents cited in the description**

- JP 2012115254 A
- WO 2015072164 A
- WO 2016027853 A
- JP 6029102 B
- JP 5870408 B

**Non-patent literature cited in the description**

- *Nature Biotechnology,* vol. 23 (7), 879-884